# EUROPEAN PATENT APPLICATION

(11) **EP 1 835 034 A1**
(43) Date of publication of application: **19.09.2007**
(21) Application number: 05822767.9
(22) Date of filing: 27.12.2005
(51) Int. Cl.: C12P 17/16, C12Q 1/06, C12Q 1/26, C12Q 1/44, C12Q 1/68, G01N 21/78

(54) **SYSTEM FOR BIOSYNTHESIS OF FIREFLY LUMINESCENCE SUBSTRATES WITH NATURAL L-CYSTEINE OR DERIVATIVES THEREOF AND LUMINESCENCE SUBSTRATE SOLUTIONS CONTAINING THE SYSTEM**

(30) Priority: 28.12.2004 JP 2004379971
(71) Applicant: The University of Electro-Communications, Chufu-shi, Tokyo 1828585 (JP)
(72) Inventor: OHMIYA, Yoshihiro Kansai Center of NATIONAL INST., Osaka, 5638577 (JP); NIWA, Kazuki Kansai Center of NATIONAL INST., Osaka, 5638577 (JP); NIWA, Haruki THE UNIV. OF ELECTRO-COMMUNIC., Tokyo 1828585 (JP); NAKAMURA, Mitsuhiro THE UNIV. OF ELECTRO-COMMUNIC., Tokyo 1828585 (JP); RYUFUKU, Masayuki c/o TOYO B-NET CO., LTD., Tokyo 1040031 (JP)
(74) Representative: Smyth, Gyles Darren
(86) International application number: PCT/JP2005/023847
(87) International publication number: WO 2006/070775

(57) **Abstract**

A method for producing firefly D-luciferin or a luminescent derivative thereof **characterized by** reacting a starting material for firefly L-luciferin in the presence of esterase, which contains at least one selected from the group consisting of (1) esters of firefly L-luciferin or derivatives thereof and (2) thioesters of firefly L-luciferin or derivatives thereof. The invention enables inexpensive determination of activity of firefly luciferase, long-time stable control of luminescence, and quantitative determination of L-cysteine, cystine and ATP.

## Description

### Technical Field

The present invention relates to a method for more simply and easily producing a stable luminescence substrate of luminescent beetle luciferase [e.g., firefly luciferase, railroad worm *(Phrixothrix hirtus)* luciferase, *Pynophorus notilucus* luciferase and *Rhagophthalmus ohbai* luciferase] useful as a reporter gene for detecting a gene expression in mammals, by directly synthesizing the substrate in a reaction solution based on natural L-cysteine whose application range is broad, and methods for measuring cysteine, ATP and the gene expression.

### Background Art

A luminescence substrate in luminescent beetles is luciferin which is a compound made from quinones and D-cysteine as starting materials (Non-patent literature 1). From uptake experiments of labeled compounds injected in fireflies in vivo, it has been proved that benzoquinone and hydroquinone are incorporated in luciferin, and it has been believed that the quinones is the starting material (Non-patent literature 2) and that there is a regeneration pathway of luciferin → oxyluciferin → 2-cyano-6-hydroxybenzothiazole → luciferin (Non-patent literature 3). Meanwhile, 2'C in luciferin has been predicted to be derived from position α in cysteine (Non-patent literature 1). Based on them, findings until now are summarized in FIG. 1. Substances in synthesis pathways identified in the firefly *in vivo* are quinone (4), 2-cyano-6-hydroxybenzothiazole (3), firefly D-luciferin (1) and oxyluciferin (2). Works to link these substances are important, but no direct research has progressed. Among them, when the starting material is supposed to be 4, cysteine (5) or cysteinyl-cysteine (7) is reacted in a reaction A which initially occurs. Subsequently, the reaction B or C can be supposed. It is supposed that a benzothiazole ring (11, 12) is made inside in the reaction B or that a thiazoline ring (10) is made in the reaction C. Furthermore, the reaction C or the reaction B becomes a metabolic pathway from the reaction B or the reaction C, respectively, and subsequently, firefly luciferin (1) is biosynthesized. Conventionally, according to this scheme, firefly luciferin has been synthesized based on D-cysteine (6) which is more expensive than a natural type.

An enzyme which converts oxyluciferin (2) which is a luminescence reaction product into 2-cyano-6-hydroxybenzothiazole (3) has been discovered (Non-patent literatures 5 and 6), and by adding the enzyme, it became possible to react 2-cyano-6-hydroxybenzothiazole (3) with D-cysteine (6) to regenerate luciferin and sustain the luminescence (Patent document 1). Anyway, in order to measure an activity of luminescent beetle luciferase, firefly D-luciferin (1) is needed, and to produce this firefly D-luciferin, it is necessary to use D-cysteine (6) which is more expensive than the natural type.

Meanwhile, firefly L-luciferin synthesized from the natural type L-cysteine did not produce the luminescence when reacted with luminescent beetle (Non-patent literatures 7 and 8). However, in recent years, an opposite experimental result that firefly L-luciferin could produce light has been reported (Non-patent literature 9). Although firefly L-luciferin synthesized from L-cysteine was converted into adenylated luciferin, being racemized easily (Non-patent literature 10), but the firefly luminescence system based on L-cysteine has not been discussed sufficiently, and no reasonable explanation is available.

L-Cysteine is a protein composing amino acid in vivo and a nutritionally unessential amino acid. In homocystinuria, homocysteine can not react with serine to become cysteine, or in cystinuria, the transport of cysteine is impaired. Thus it is important to measure L-cysteine *in vivo* or measure an amount of cystine in urine. A method for measuring L-cysteine quantity by quantifying any of reaction products of an enzyme, L-cysteine degrading enzyme, in the presence of pyridoxal-5'-phosphate, in which the enzyme produces ammonia, pyruvic acid and hydrogen sulfide from L-cysteine, but not from D-cysteine and homocysteine, is available(Patent document 2). Also, a reagent for measuring L-cysteine quantity, which comprises an enzyme which produces ammonia, pyruvic acid and hydrogen sulfide from L-cysteine, but not from D-cysteine and homocysteine, and pyridoxal-5'-phosphate is available(Patent document 2). On the other hand, there is a D-cysteine quantification method, which comprises characteristic two steps: in the first step, D-cysteine is reacted with a D-luciferin precursor or D-luciferin precursor derivatives (2-cyano-6-hydroxybenzothiazole, 2-cyano-6-o-0-D-galactosyl benzothiazole, and so on) to produce D-luciferin or D-luciferin derivatives, and in the second step, the resultant D-luciferin or D-luciferin derivatives is quantified to measure the amount of D-cysteine (Patent document 3).
Patent document 1: JP 2001-103972-A;
Patent document 2: JP 2004-105057-A;
Patent document 3: JP 2000-275247-A;
Non-patent literature 1: White E. H., McCapra F., Fireld G. and McElroy W. D. (1961) J. Amer. Chem. Soc., 83, 2402-2403;
Non-patent literature 2: Okada, K., Ito H. & Goto T. (1976) J. C. S. Chem. Comm. , 32;
Non-patent literature 3: Okada, K., Ito H., Kubota I. & Goto T. (1974) Tetrahedron Letters, 15, 2771-2774;
Non-patent literature 4: McCapra, F. & Razavi, Z. (1976) J. C. S. Chem. Comm., 153-154;
Non-patent literature 5: Gomi, K. & Kajiyama, N. (2001), J. Biol. Chem., 276, 36508-36513;
Non-patent literature 6: Gomi, K., Hirokawa, K., Kajiyama, N. (2002), Gene 294, 157-66;
Non-patent literature 7: White E. H.; McCapra F.; Fireld G.; McElroy W. D. (1961), J. Amer. Chem. Soc., 83, 2402-2403;
Non-patent literature 8: E. H. White, F. McCapra and G. F. Field. (1963), J. Amer. Chem. Soc., 85, 337-343;
Non-patent literature 9: Lembert, N. (1996), Biochem. J., 317, 273-7;
Non-patent literature 10: Imai, K.; Goto, T. (1988), Agric. Biol. Chem., 52, 2803-2809.

### DISCLOSURE OF THE INVENTION

### PROBLEMS TO BE SOLVED BY THE INVENTION

It is an object of the present invention to develop a method for yielding the luminescence of firefly luciferase which is useful as a reporter gene for detecting gene expression in mammals, without directly using the firefly luminescence substrate, based on more simply and easily a method to produce stable substrate in the reaction solution from natural L-cysteine or the derivatives of L-cysteine whose application range is broad, as well as a method for measuring quantity of natural cysteine, cysteine derivatives including cystine, ATP, and the gene expression. This enables to measure an activity of firefly luciferase inexpensively, control the stable luminescence over a long time and quantify cysteine, cysteine derivatives including cystine, ATP, and the gene expression.

### MEANS FOR SOLVING THE PROBLEMS

For solving the above problems, the present inventor reconfirmed biosynthesis pathways of the firefly luminescence substrate, discovered a potential pathway for the luminescence substrate which was different from those previously received, and has found that a luminescence substrate is biosynthesized starting with natural L-cysteine in the absence of firefly D-luciferin, and the luminescence is produced by adding the substances involved in that pathway together with firefly luciferase. Thus, the present inventor studied methods for stably and simply purifying the luminescence substrate in a short period as well as a storage method, and completed the present invention.

The present invention provides the following methods for synthesizing the firefly luminescence substrate, luminescence substrate solution for reporter assay, and methods for measuring ATP and cysteine.
[1] A method for producing firefly D-luciferin and a luminescent derivative thereof characterized by reacting starting materials for firefly L-luciferin in the presence of esterase, which contains at least one selected from the following two groups: (1) esters of firefly L-luciferin or derivatives thereof and (2) thioesters of firefly L-luciferin or derivatives thereof.
[2] The method according to [1] wherein the starting material for firefly L-luciferin starting material is firefly L-luciferyl-CoA or the racemic modification thereof.
[3] The method according to [1] wherein the esterase is derived from a mammal or an insect.
[4] The method according to [3] wherein the esterase derived from the insect is firefly esterase.
[5] A method for producing firefly D-luciferin or a luminescent derivative thereof characterized by reacting firefly L-luciferin or a firefly L-luciferin derivative or a supply source thereof with luciferase in the presence of coenzyme A, ATP and Mg²⁺ to yield firefly L-luciferyl-CoA, and reacting the firefly L-luciferyl-CoA with esterase.
[6] The method according to [5] wherein the supply source of the firefly L-luciferin derivative is composed of L-cysteine or an L-cysteine derivative and a compound represented by the following formula (1): 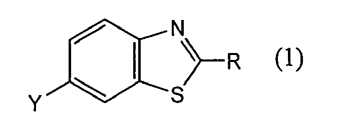 wherein Y represents a hydroxyl group, an alkoxy group, an acyloxy group, an amino group, a monoalkylamino group, dialkylamino group or a group: 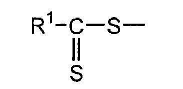 (wherein R¹ represents an alkyl group, an aralkyl group or aryl group), and R represents CN or a group: 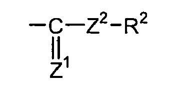 (wherein R² represents an alkyl group, an aralkyl group or aryl group; and Z¹ and Z² are the same or different and represent oxygen (O) atoms or sulfur (S) atoms).
[7] A system for measuring a luminescence activity of firefly luciferase or luminescent derivatives thereof, comprising firefly L-luciferin or a derivative thereof or a supply source thereof, coenzyme A, ATP, Mg²⁺, and esterase.
[8] A method for quantifying L-cysteine or a derivative thereof characterized by measuring a luminescence produced from firefly D-luciferin or a luminescent derivative thereof in a reaction system comprising a compound represented by the following formula (1): 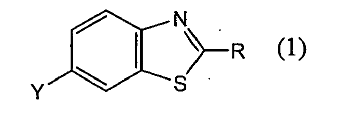 wherein Y represents a hydroxyl group, an alkoxy group, an acyloxy group, an amino group, a monoalkylamino group, dialkylamino group or a group: 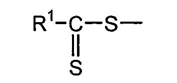 (wherein R¹ represents an alkyl group, an aralkyl group or aryl group), and R represents CN or a group: 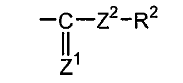 (wherein R² represents an alkyl group, an aralkyl group or aryl group; and Z¹ and Z² are the same or different and represent oxygen (O) atoms or sulfur (S) atoms), coenzyme A, ATP, Mg²⁺, luminescent beetle luciferase and esterase.
[9] A method for quantifying ATP characterized by measuring a luminescence produced from firefly D-luciferin or a luminescent derivative thereof in a reaction system comprising firefly L-luciferin or a derivative thereof or a supply source thereof, coenzyme A, Mg²⁺, luminescent beetle luciferase and esterase.
[10] A method for quantifying microorganisms characterized by measuring a luminescence produced from firefly D-luciferin or a luminescent derivative thereof in a reaction system comprising firefly L-luciferin or a derivative thereof or a supply source thereof, coenzyme A, Mg²⁺, luminescent beetle luciferase and esterase.
[11] A method for quantifying esterase characterized by measuring a luminescence produced from firefly D-luciferin or a luminescent derivative thereof in a reaction system comprising firefly L-luciferin or a derivative thereof or a supply source thereof, coenzyme A, ATP, Mg²⁺ and luminescent beetle luciferase.
[12] A kit for quantifying L-cysteine or a derivative thereof comprising a compound represented by the following formula (1): 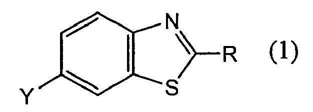 wherein. Y represents a hydroxyl group, an alkoxy group, an acyloxy group, an amino group, a monoalkylamino group, dialkylamino group or a group: 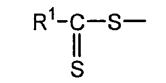 (wherein R¹ represents an alkyl group, an aralkyl group or aryl group), and R represents CN or a group: 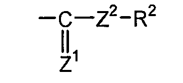 (wherein R² represents an alkyl group, an aralkyl group or aryl group; and Z¹ and Z² are the same or different and represent oxygen (O) atoms or sulfur (S) atoms), luminescent beetle luciferase, coenzyme A, ATP, Mg²⁺ and esterase.
[13] A kit for quantifying ATP comprising firefly L-luciferin or a derivative thereof or a supply source thereof, luminescent beetle luciferase, esterase, coenzyme A and Mg²⁺.
[14] A kit for quantifying microorganisms comprising firefly L-luciferin or a derivative thereof or a supply source thereof, luminescent beetle luciferase, esterase, coenzyme A and Mg²⁺.
[15] A kit for quantifying esterase comprising firefly L-luciferin or a derivative thereof or a supply source thereof, luminescent beetle luciferase, coenzyme A, ATP and Mg²⁺.

### EFFECTS OF THE INVENTION

In the present invention, the pathway to synthesize firefly D-luciferin capable of producing the luminescence using L-cysteine as a starting material has been found, and by utilizing this, it is possible to construct a luminescence substrate reagent, a cysteine quantifying reagent and a reagent for measuring an esterase activity. This enables to measure the activity of firefly luciferase inexpensively, control the stable luminescence over a long time and quantify cysteine, cystine and ATP.

### BRIEF DESCRIPTION OF THE DRAWINGS

FIG 1 is a view showing a scheme of biosynthesis pathways of firefly luciferin;
FIG. 2 is a graph showing a correlation between luciferase concentrations and luminescence amounts in an L-cysteine-based luminescence measuring reagent;
FIG. 3 is a view showing an HPLC profile for separation of firefly D-luciferin from firefly L-luciferin, and a reaction scheme of firefly D-luciferin or firefly L-luciferin synthesized from D-cysteine or L-cysteine with 2-cyano-6-hydroxybenzothiazole;
FIG. 4 is a view showing a scheme in which firefly D-luciferin is synthesized from L-cysteine and 2-cyano-6-hydroxybenzothiazole with esterase via luciferyl-CoA in the presence of luciferase;
FIG. 5 is a graph showing a correlation between swine esterase concentrations and luminescence amounts in an L-cysteine-based luminescence measuring reagent;

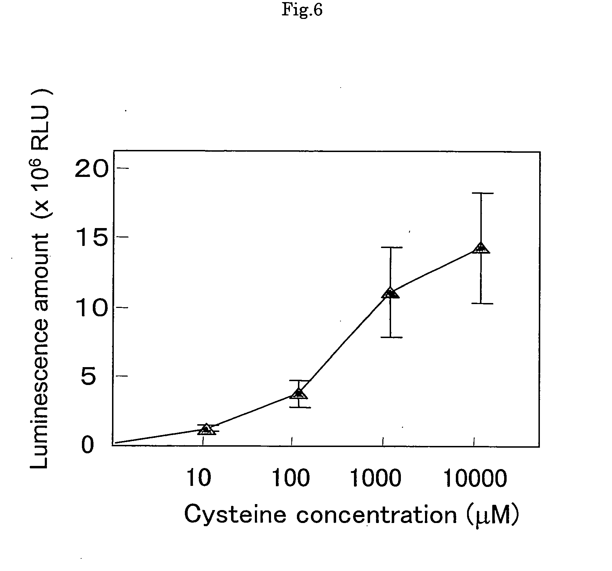

FIG. 6 is a graph showing a correlation between L-cysteine concentrations and luminescence amounts in an L-cysteine-based luminescence measuring reagent; and
FIG. 7 is a graph showing a correlation between ATP concentrations and luminescence amounts in an L-cysteine-based luminescence measuring reagent;

### BEST MODES FOR CARRYING OUT THE INVENTION

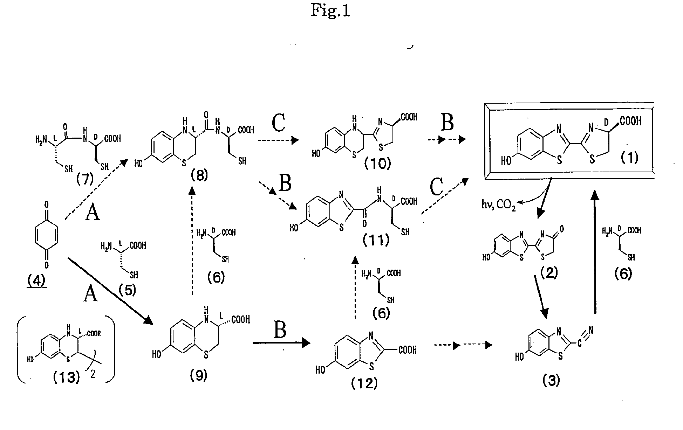

A firefly luminescence system is used for measuring a reporter enzyme for analyzing the gene expression (quantification of luminescent beetle luciferase such as firefly luciferase), for measuring amounts of ATP which is a cofactor of the reaction or for industrial uses such as detection of bacteria using the ATP amount as an indicator, and many manufactured articles have been produced. In a reaction mechanism of this firefly luminescence system, firefly D-luciferin which is a luminescence substrate reacts with ATP to become adenyl-luciferin, which subsequently reacts with an enzyme to become oxyluciferin, and at that time, the luminescence occurred. At that time, luminescent beetle luciferase such as firefly luciferase as a catalyst facilitates the reaction and further magnesium ion is required as a cofactor. Substances essentially required for producing the luminescence reaction are firefly D-luciferin, ATP, the magnesium ion and luminescent beetle luciferase (in particular, firefly luciferase). At that time, it is believed that luminescent beetle luciferase recognizes a chirality of luciferin, and that the luminescence is not produced by a non-natural type firefly L-luciferin and can be produced only by firefly D-luciferin. Therefore, when firefly D-luciferin is biosynthesized, it has been believed that this substrate is synthesized from D-cysteine (FIG. 1).

However, it has been found that when L-cysteine and 2-cyano-6-hydroxybenzothiazole are mixed, firefly L-luciferin having no luminescence activity is synthesized, and further when coenzyme A, ATP, Mg ion and thioester hydrolase (esterase) are added, firefly D-luciferin capable of producing the luminescence is synthesized via L-luciferyl-CoA. This has revealed the system in which firefly D-luciferin capable of producing the luminescence is efficiently synthesized from natural L-cysteine or a D- and L-cysteine mixture (particularly a racemic modification) in which a portion of D-cysteine is contained in reaction reagents, and which does not require enatiomeric pure D-cysteine as the starting material.

In one embodiment of the present invention, the starting material for producing firefly D-luciferin or a luminescent derivative thereof includes at least one selected from the group consisting of esters and thioesters of firefly L-luciferin or derivative thereof. In these starting materials, (thio)ester is hydrolyzed by an action of (thio)esterase, and at that time, their configuration is reversed to form firefly D-luciferin or the luminescent derivative thereof.

The derivative of firefly L-luciferin includes those having an alkoxy, acyloxy, amino, monoalkylamino or dialkylamino substituent at position 6 of a benzothiazole group.

Ester of firefly L-luciferin or the derivative thereof includes C₁ to C₆ straight or branched alkyl ester such as methyl ester, ethyl ester, n-propyl ester, isopropyl ester, t-butyl ester, pentyl ester and hexyl ester; aralkyl ester such as benzyl ester; and aryl ester such as phenyl ester.

Thioester of firefly L-luciferin or the derivative thereof includes C₁ to C₆ straight or branched alkyl thioester such as methyl thioester, ethyl thioester, n-propyl thioester, isopropyl thioester, t-butyl thioester, pentyl thioester and hexyl thioester; and ester of coenzyme A (sometimes abbreviated as CoA or CoA-SH) or a partial structure of coenzyme A having SH group, e.g., cysteamine and amide of pantothenic acid and cysteamine.

The luminescent derivative of firefly D-luciferin includes derivatives having an acyloxy, alkoxy, amino, monoalkylamino, dialkylamino group or a group: 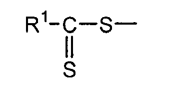 (wherein R¹ is the same as defined above) at position 6 of the benzothiazole group.

In one embodiment of the present invention, L-firefly luciferyl (thio)ester such as firefly L-luciferyl-CoA is converted into firefly D-luciferin when degraded using esterase.

In order to convert firefly L-luciferin into firefly D-luciferin, it is preferable to use thioester of firefly L-luciferin, and it is more preferable to use ester of coenzyme A or the partial structure of coenzyme A such as cysteamine involved in thioester formation.

As the enzyme which hydrolyzes ester of firefly L-luciferin, both esterase and thioesterase can be used. Both esterase and thioesterase can be used for the hydrolysis of ester of firefly L-luciferin. Likewise, esterase can also be used for the hydrolysis of thioester of firefly L-luciferin.

Esterase which can hydrolyze thioester includes, for example esterase derived from mammal such as swines, human beings, sheeps, dogs, monkeys, mice, rats, hamsters and guinea pigs, and esterase derived from insects such as firefly, *Pynophorus notilucus,* and silk worm.

It is preferable that CoA ester of firefly L-luciferin is hydrolyzed with esterase because the configuration is efficiently converted.

Ester and thioester of firefly L-luciferin may be L-isomer at 100% or the mixture of D- and L-isomers in which a portion is the D-isomer (corresponding to firefly D-luciferin). A mixed ratio of the D-isomer in the starting material ester/thioester is typically 50% or less, and may be exceeding 50%.

The supply source of firefly L-luciferin or the derivative thereof is composed of L-cysteine or the L-cysteine derivative and the compound of the following formula (1): 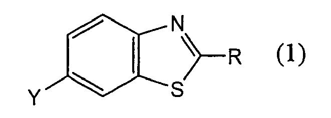 wherein Y and R are the same as defined above. The L-cysteine derivative includes L-cysteine ester (e.g., alkyl ester, aralkyl ester, aryl ester). As the alkyl, aralkyl and aryl groups in alkyl ester, aralkyl ester and aryl ester of L-cysteine, those which are the same as the above are exemplified.

In the present invention, the alkoxy group includes C₁ to C₆ straight or branched alkoxy groups such as methoxy, ethoxy, n-propoxy, isopropoxy, n-butoxy, isobutoxy, sec-butoxy, t-butoxy, pentoxy and hexyloxy.

The acyloxy group includes formyloxy, acetyloxy, propionyloxy, n-butyryloxy, sec-butyryloxy, tert-butyryloxy, valeryloxy, isovaleryloxy, pivaloyloxy, glycolyloxy, lactoyloxy, phenylacetyloxy and benzoyloxy.

The monoalkylamino group includes amino groups substituted with C₁ to C₆ preferably C₁ to C₄ straight or branched alkyl, such as methylamino, ethylamino, n-propylamino, isopropylamino, n-butylamino, isobutylamino, sec-butylamino, tert-butylamino, pentylamino and hexylamino.

The dialkylamino group includes amino groups di-substituted with C₁ to C₆ preferably C₁ to C₄ straight or branched alkyl, such as dimethylamino, diethylamino, di-n-propylamino, diisopropylamino, di-n-butylamino, diisobutylamino, di-sec-butylamino, di-tert-butylamino, dipentylamino and dihexylamino.

The alkyl group includes C₁ to C₆ preferably C₁ to C₄ straight or branched alkyl groups such as methyl, ethyl, n-propyl, isopropyl, n-butyl, isobutyl, sec-butyl, tert-butyl, pentyl and hexyl.

The aralkyl group includes benzyl, 1-phenethyl and 2-phenethyl.

The aryl group includes phenyl, naphthyl, tolyl and xylyl.

One embodiment of the present invention is the firefly luminescence system incorporating firefly L-luciferin or the derivative thereof or the supply source thereof, and coenzyme A, ATP, the Mg ion, luminescent beetle luciferase and esterase.

One preferable embodiment of the present invention is the firefly luminescence system incorporating L-cysteine, 2-cyano-6-hydroxybenzothiazole and coenzyme A, ATP, the Mg ion, firefly luciferase and esterase.

One characteristic of the present invention is in that D-luciferin is obtained by hydrolyzing firefly L-luciferin ester (preferably thioester) such as firefly L-luciferyl-CoA to reverse the configuration of the carboxyl group in L-luciferin in the presence of esterase which can hydrolyze the esterase.

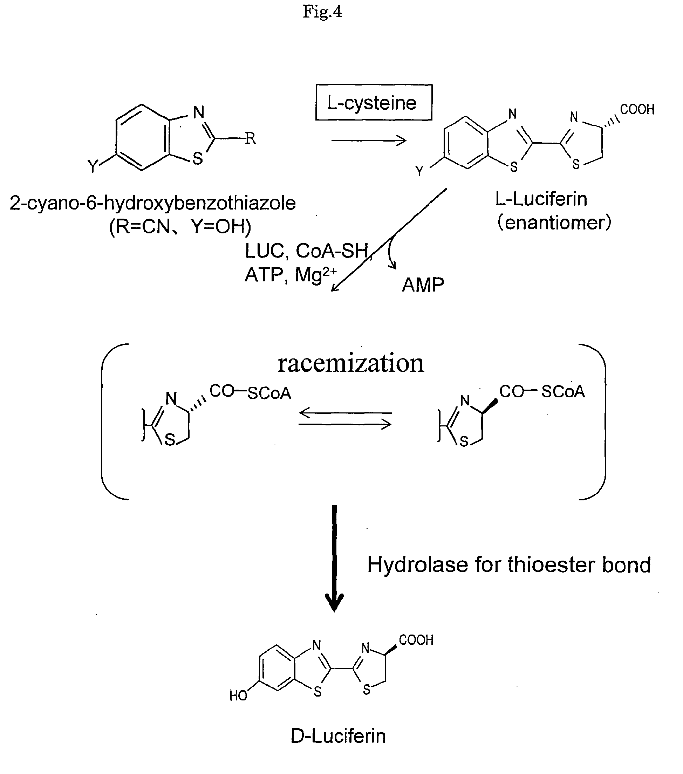

L-luciferyl-CoA which is particularly preferable as the starting material for producing D-luciferin can be obtained by reacting L-luciferin and coenzyme A (sometimes abbreviated as CoA-SH; CoA), ATP, Mg²⁺ and firefly luciferase (LUC) as shown in FIG. 4. The reaction for producing firefly L-luciferin is advantageously carried forward by reacting L-cysteine and 2-cyano-6-hydroxybenzothiazole in an appropriate solvent such as water, alcohol. e.g., ethanol, methanol or propanol, or aqueous alcohol, acetonitrile, acetone THF, DMF or DMSO, or alternatively in a two phase system of water-organic solvent at room temperature to the temperature at which the solvent is boiled for about one to 24 hours.

According to the luminescence system of the present invention, a reagent system based on inexpensive and natural L-cysteine can be constructed without using expensive and non-natural D-cysteine and firefly D-luciferin.

In the luminescence system of the present invention, the luminescence amount is linearly correlated with the firefly luciferase amount, the L-cysteine amount, the ATP amount and the amount of thioester hydrolase. Thus, the reagent for detecting the gene expression, the reagent for quantifying L-cysteine, the reagent for quantifying ATP and the reagent for measuring the esterase activity can be constructed using this luminescence system. By quantifying ATP, it is also possible to quantify the bacteria.

In a measurement system of the present invention, by quantifying the amount of luminescence produced by D-luciferin obtained from L-luciferin or the supply source thereof (L-cysteine or the derivative thereof and 2-cyano-6-hydroxybenzothiazole) by being catalyzed with ATP and luciferase, it is possible to quantify ATP, L-cysteine or the derivative thereof, luciferase and esterase involved in the measurement system.

In one preferable embodiment of the present invention, the concentration of each component used in the measurement system is as follows.
ATP: 0.1 to 10 mM, preferably 1 to 5 mM
Firefly luciferase: 0.0001 to 10 units/mL, preferably 0.1 to 1 unit/mL
Esterase: 10⁻⁶ to 10⁻² units/mL, preferably 10⁻⁴ to 10⁻³ units/mL L-cysteine or the derivative thereof: 0.01 to 20 mM, preferably 0.1 to 10 mM.

The temperature upon measurement is preferably the room temperature to about 37°C, and a measurement time period is preferably about one to 50 seconds.

In the luminescence system of the present invention, firefly L-luciferin is converted into firefly D-luciferin by esterase such as thioester hydrolase. By combining an antioxidant, its storage stability is enhanced. The antioxidant includes ascorbic acid and deoxidizers.

### EXAMPLES

The present invention will be described in more detail based on the following Examples.

### Example 1

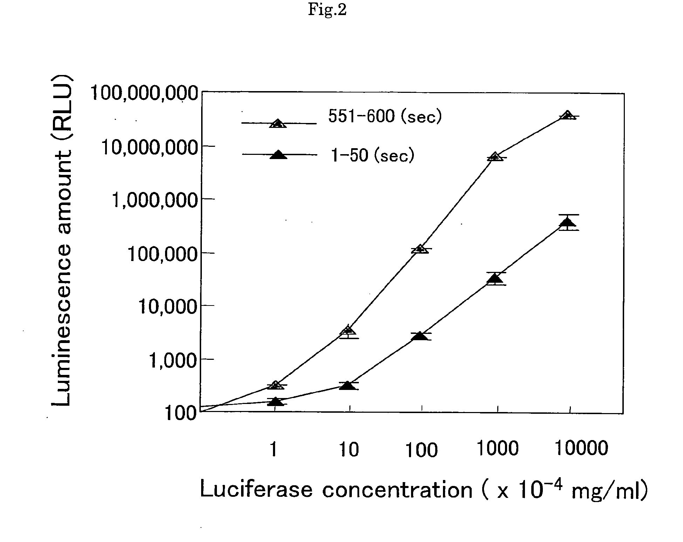

In a buffer solution (0.1 M Tris-HCl, pH 8). 10 mM 2-cyano-6-hydroxybenzothiazole, 1 mM L-cysteine, 10 mM CoA-SH, 0.1 mg/mL of firefly luciferase and 100 units/mL of swine liver esterase were dissolved, 10 µL of the mixture was dispensed in a tube for measuring the luminescence, and a total volume was made 100 µL by adding the same buffer. An ATP-Mg solution (100 µL, 6 mM ATP-Na, 16 mM MgSO₄) was injected thereto to initiate a luminescence reaction, and a luminescence amount was measured using a luminometer AB2200 supplied from ATTO Corporation. As a result, the luminescence was identified. As shown in FIG. 2, the luminescence amount was measured by changing the luciferase concentration from 1 x 10⁻⁴ mg/mL to 1 mg/mL. As a result, it was found that an accumulated luminescence intensity for one to 50 seconds and for 551 to 600 seconds after the initiation was linearly changed in correlation with the concentration of the luminescence enzyme. Therefore, it was shown that the luminescence reaction occurred if the luminescence enzyme, luciferase was present in the presence of L-cysteine, 2-cyano-6-hydroxybenzothiazole, CoA-SH and esterase. This has revealed that the luminescence substrate is synthesized from L-cysteine under these conditions even if the firefly D-luciferin which is the luminescence substrate is not added. In addition, it has been revealed that the luminescence activity depends on the amount of the luminescence enzyme and that the luminescence enzyme can be quantified by measuring the amount of the luminescence activity.

### Example 2

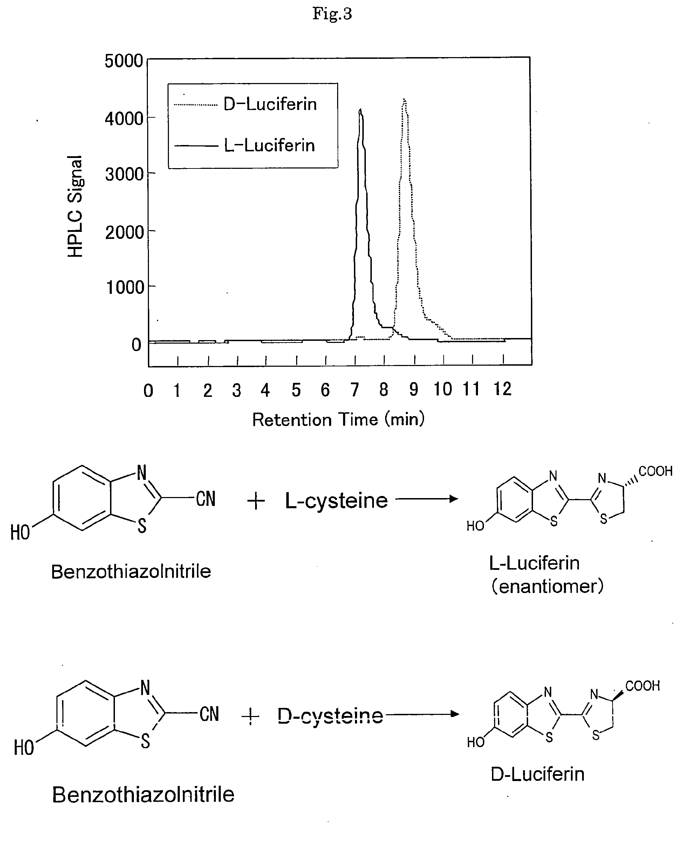

FIG. 3 is a signal profile at a fluorescence wavelength of 530 nm obtained by mixing D-cysteine or L-cysteine with 2-cyano-6-hydroxybenzothiazole at room temperature, subsequently separating by HPLC and exciting at an excitation wavelength of 330 nm. The separation was performed using Chiralcel OD-RH supplied from Daicel Chemical Industries, Ltd., and a 27% acetonitrile and 0.1% TFA (trifluoroacetic acid) aqueous system as a separation solvent at a flow rate of 1.0 mL/minute. When previously synthesized firefly D-luciferin and firefly L-luciferin were separated, firefly L-luciferin and firefly D-luciferin were eluted at retention times of 7 minutes and 9 minutes, respectively. When the mixture was separated, each luciferin was eluted at the same retention time, identifying that 2-cyano-6-hydroxybenzothiazole reacted with D-cysteine to generate firefly D-luciferin and reacted with L-cysteine to generate firefly L-luciferin, respectively. It was also identified by mass spectrum that luciferyl-CoA was present under the condition where 10 mM nitrile, 1 mM L-cysteine, 10 mM CoA-SH and 0.1 mg/mL of luciferase had been added. From these results, the pathway where firefly D-luciferin was synthesized from L-cysteine as shown in FIG. 4 was identified. That is, firefly L-luciferin synthesized from L-cysteine and 2-cyano-6-hydroxybenzothiazole becomes luciferyl-CoA in the presence of luciferase. This product does not produce the luminescence, but swine liver esterase converts this product into firefly D-luciferin which produces the luminescence.

### Example 3

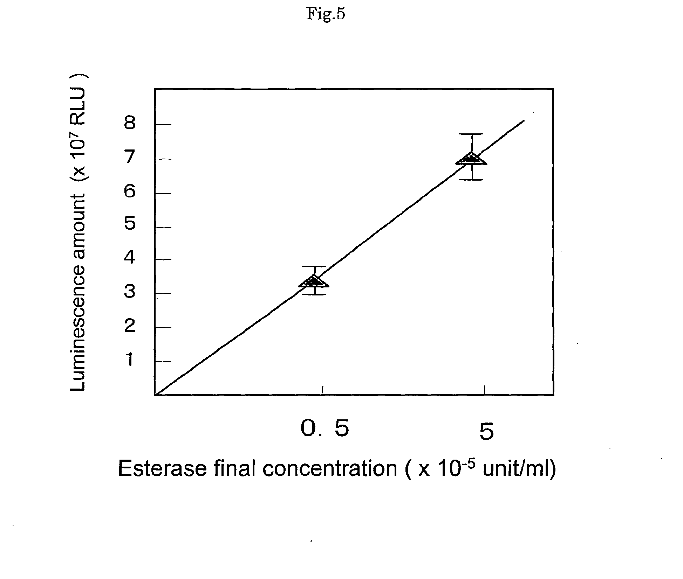

In the buffer solution (0.1 M Tris-HCl, pH 8), 10 mM 2-cyano-6-hydroxybenzothiazole, 1 mM L-cysteine, 10 mM CoA-SH, 0.1 mg/mL of firefly luciferase and 100 units/mL or 10 unite/mL of swine liver esterase were dissolved, 10 µL of the mixture was dispensed in the tube for measuring the luminescence, and the total volume was made 100 µL by adding the same buffer. The ATP-Mg solution (100 µL, 6 mM ATP-Na, 16 mM MgSO₄) was injected thereto to initiate the luminescence reaction, and the luminescence amount was measured using the luminometer AB2200 supplied from ATTO Corporation. FIG. 5 shows the relation between the esterase amount and the luminescence activity, and when the esterase concentration was doubled, the luminescence amount was also doubled. Thus, it has been revealed that a conversion efficiency of L-cysteine and 2-cyano-6-hydroxybenzothiazole→ firefly L-luciferin → firefly D-luciferin depends on the amount of swine liver esterase.

### Example 4

In the buffer solution (0.1 M Tris-HCl, pH 8), 10 mM 2-cyano-6-hydroxybenzothiazole, 100 to 0.1 mM L-cysteine, 10 mM CoA-SH, 0.1 mg/mL of firefly luciferase and 100 units/mL of swine liver esterase were dissolved, 10 µL of the mixture was dispensed in the tube for measuring the luminescence, and the total volume was made 100 µL by adding the same buffer. The ATP-Mg solution (100 µL, 6 mM ATP-Na, 16 mM MgSO₄) was injected thereto to initiate the luminescence reaction, and the luminescence amount was measured using the luminometer AB2200 supplied from ATTO Corporation. FIG, 6 shows the relation between L-cysteine and the luminescence activity, and when the concentration of L-cysteine was changed from 10 to 10000 µM, the luminescence activity was nearly linearly increased. Thus, it has been revealed that the conversion efficiency of L-cysteine and 2-cyano-6-hydroxybenzothiazole → firefly L-luciferin → firefly D-luciferin depends on L-cysteine, and that L-cysteine can be quantified by measuring the luminescence activity.

### Example 5

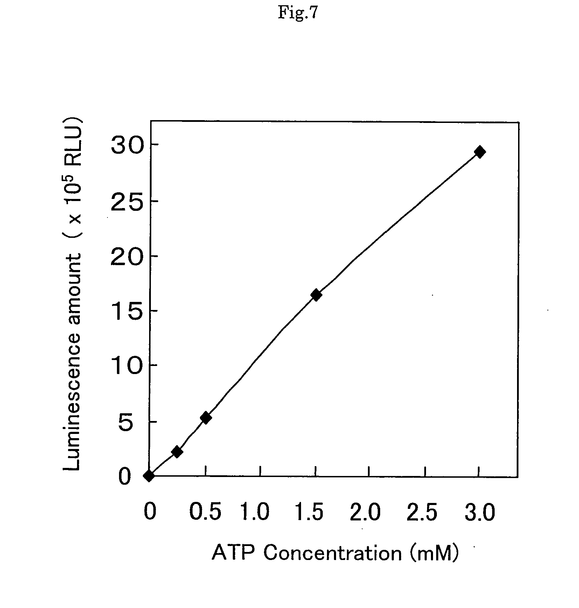

In the buffer solution (0.1 M Tris-HCl, pH 8), 10 mM 2-cyano-6-hydroxybenzothiazole, 1 mM L-cysteine, 10 mM CoA-SH, 0.1 mg/mL of firefly luciferase and 100 units/mL of swine liver esterase were dissolved, 10 µL of the mixture was dispensed in the tube for measuring the luminescence, and the total volume was made 100 µL by adding the same buffer. The ATP-Mg solution (100 µL, 0.5 to 6 mM ATP-Na, 16 mM MgSO₄) was injected thereto to initiate the luminescence reaction, and the luminescence amount was measured using the luminometer AB2200 supplied from ATTO Corporation. FIG. 7 shows the relation between the ATP amount and the luminescence activity, and when the ATP concentration was changed from 0.25 to 3 mM, the luminescence activity was nearly linearly increased. Thus, it has been revealed that the conversion efficiency of L-cysteine and 2-cyano-6-hydroxybenzothiazole → firefly L-luciferin → firefly D-luciferin depends on the ATP amount, and that ATP can be quantified by measuring the luminescence activity.

## Claims

1. A method for producing firefly D-luciferin and a luminescent derivative thereof **characterized by** reacting starting materials for firefly L-luciferin in the presence of esterase, which contains at least one selected from selected from the following two groups: (1) esters of firefly L-luciferin or derivatives thereof and (2) thioesters of firefly L-luciferin or derivatives thereof.

2. The method according to claim 1 wherein said firefly L-luciferin starting materials are firefly L-luciferyl-CoA or a racemic modification thereof.

3. The method according to claim 1 wherein said esterase is derived from a mammal or an insect.

4. The method according to claim 3 wherein the esterase derived from the insect is firefly esterase.

5. A method for producing firefly D-luciferin or a luminescent derivative thereof **characterized by** reacting firefly L-luciferin or a firefly L-luciferin derivative or a supply source thereof with luciferase in the presence of coenzyme A, ATP and Mg²⁺ to yield firefly L-luciferyl-CoA, and reacting the firefly L-luciferyl-CoA with esterase.

6. The method according to claim 5 wherein the supply source of firefly L-luciferin or the derivative thereof is composed of L-cysteine or an L-cysteine derivative and a compound represented by the following formula (1): 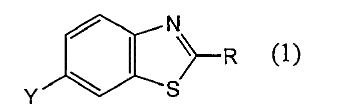 wherein Y represents a hydroxyl group, an alkoxy group, an acyloxy group, an amino group, a monoalkylamino group, dialkylamino group or a group: 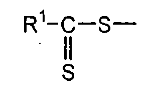 (wherein R¹ represents an alkyl group, an aralkyl group or aryl group), and R represents CN or a group: 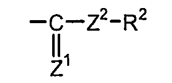 (wherein R² represents an alkyl group, an aralkyl group or aryl group; and Z¹ and Z² are the same or different and represent oxygen (O) atoms or sulfur (S) atoms).

7. A system for measuring a luminescence activity of firefly luciferase or luminescent derivatives thereof, comprising firefly L-luciferin or a derivative thereof or a supply source thereof, coenzyme A, ATP, Mg²⁺, and esterase.

8. A method for quantifying L-cysteine or a derivative thereof **characterized by** measuring a luminescence produced from firefly D-luciferin or a luminescent derivative thereof in a reaction system comprising a compound represented by the following formula (1) : 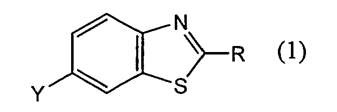 wherein Y represents a hydroxyl group, an alkoxy group, an acyloxy group, an amino group, a monoalkylamino group, dialkylamino group or a group: 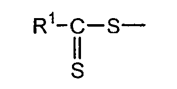 (wherein R¹ represents an alkyl group, an aralkyl group or aryl group), and R represents CN or a group: 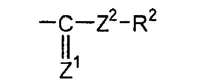 (wherein R² represents an alkyl group, an aralkyl group or aryl group; and Z¹ and Z² are the same or different and represent oxygen (O) atoms or sulfur (S) atoms), coenzyme A, ATP, Mg²⁺, luminescent beetle luciferase and esterase.

9. A method for quantifying ATP **characterized by** measuring a luminescence occurring from firefly D-luciferin or a luminescent derivative thereof in a reaction system comprising firefly L-luciferin or a derivative thereof or a supply source thereof, coenzyme A, Mg²⁺, luminescent beetle luciferase and esterase.

10. A method for quantifying microorganisms **characterized by** measuring a luminescence produced from firefly D-luciferin or a luminescent derivative thereof in a reaction system comprising firefly L-luciferin or a derivative thereof or a supply source thereof, coenzyme A, Mg²⁺, luminescent beetle luciferase and esterase.

11. A method for quantifying esterase **characterized by** measuring a luminescence produced from firefly D-luciferin or a luminescent derivative thereof in a reaction system comprising firefly L-luciferin or a derivative thereof or a supply source thereof, coenzyme A, ATP, Mg²⁺ and luminescent beetle luciferase.

12. A kit for quantifying L-cysteine or a derivative thereof comprising a compound represented by the following formula (1): 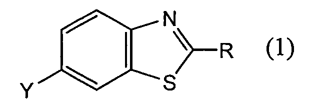 wherein Y represents a hydroxyl group, an alkoxy group, an acyloxy group, an amino group, a monoalkylamino group, dialkylamino group or a group: 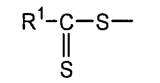 (wherein R¹ represents an alkyl group, an aralkyl group or aryl group), and R represents CN or a group: 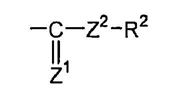 (wherein R² represents an alkyl group, an aralkyl group or aryl group; and Z¹ and Z² are the same or different and represent oxygen (O) atoms or sulfur (S) atoms), luminescent beetle luciferase, coenzyme A, ATP, Mg²⁺ and esterase.

13. A kit for quantifying ATP comprising firefly L-luciferin or a derivative thereof or a supply source thereof, luminescent beetle luciferase, esterase, coenzyme A and Mg²⁺.

14. A kit for quantifying microorganisms comprising firefly L-luciferin or a derivative thereof or a supply source thereof, luminescent beetle luciferase, esterase, coenzyme A and Mg²⁺.

15. A kit for quantifying esterase comprising firefly L-luciferin or a derivative thereof or a supply source thereof, luminescent beetle luciferase, coenzyme A, ATP and Mg²⁺.
